# EUROPÄISCHE PATENTSCHRIFT

(11) **EP 1 019 037 B1**
(45) Veröffentlichungstag und Bekanntmachung des Hinweises auf die Patenterteilung: **08.08.2001**
(21) Anmeldenummer: 98952606.6
(22) Anmeldetag: 21.09.1998
(51) Int. Cl.: A61K 31/195, A61K 47/02

(54) **DELTA-AMINOLÄVULINSÄURE ENTHALTENDES ARZNEIMITTEL**
MEDICAMENT CONTAINING DELTA-AMINOLEVULINIC ACID
MEDICAMENT CONTENANT DE L'ACIDE DELTA-AMINOLEVULIQUE

(30) Priorität: 02.10.1997 DE 19744811
(43) Veröffentlichungstag der Anmeldung: 19.07.2000
(73) Patentinhaber: Medac Gesellschaft für klinische Spezialpräparate mbH, D-20354 Hamburg (DE)
(72) Erfinder: HEISIG, Wolfgang, D-22395 Hamburg (DE); BACH, Ferdinand, D-22880 Wedel (DE)
(74) Vertreter: UEXKÜLL & STOLBERG
(86) Internationale Anmeldenummer: EP9805979
(87) Internationale Veröffentlichungsnummer: WO9917764

(56) Entgegenhaltungen:
- EP-A- 0 358 234
- WO-A-95/05813
- WO-A-95/07077
- DE-A- 4 228 106
- FR-A- 2 690 340

## Beschreibung

Die Erfindung betrifft die Verwendung von δ-Aminolävulinsäure (ALA) zur Herstellung eines topischen Arzneimittels zur integralen Diagnose und/oder Therapie von Tumoren in Hohlorganen.

Bösartige Tumor-Erkrankungen in Hohlorganen, wie zum Beispiel Harn- und Atemwege, Peritonealhöhle, Gastrointestinaltrakt und weiblicher Genitialtrakt, zeichnen sich durch hohe Rezidiv- und Progressionsraten aus. Diese sind Ausdruck bereits synchron vorliegender präkanzeröser und kanzeröser Veränderungen (Frühkarzinome), die endoskopisch nicht diagnostizierbar und damit im frühen Stadium nicht therapierbar sind.

Bei Frühkarzinomen der Harnblase beispielsweise betragen die Rezidiv- und Progressionsraten bis zu 90 %. Eine potentielle Entartung im Bereich der gesamten Blasenschleimhaut ist daher zu postulieren. Die präkanzerösen und kanzerösen Veränderungen, wie zum Beispiel schwere Urotheldysplasien, Carcinoma in situ und multifokale mikropapilläre Tumore, können endskopisch nicht oder nur sehr schwer erkannt werden.

Für eine kurative Behandlung ist zum einen Voraussetzung, sämtliche Tumorherde umfassend zu erkennen, zum anderen ist eine vollständige und integrale Behandlung der gesamten Blasenschleimhaut gefordert.

Keines der bislang bekannten diagnostischen Verfahren ist in der Lage, Tumorherde auch im Frühstadium vollständig und gezielt zu erkennen. Für die Therapie der Blasenschleimhaut werden bislang Medikamente (Zytostatika, BCG) in die Harnblase instilliert. Zwar können die Rezidivintervalle auf diese Weise verlängert werden, eine endgültige Heilung ist im Rahmen der bislang bekannten Therapieverfahren jedoch nicht möglich (J. Urol. 1987, 138, 1363-1368).

Alternativ zu den konservativen Diagnose- und Therapiemethoden wird die Photodynamik eingesetzt. Sie beruht auf der Lichtabsorption durch eine chemische Verbindung, den sogenannten Photosensibilisator, was einerseits zu einer Fluoreszenzemission führt, die zur Diagnose ausgenutzt werden kann und andererseits eine zytotoxische Reaktion hervorruft, die für die Therapie von Nutzen ist.

Im Rahmen der Photodynamik werden gegenwärtig als Photosensibilisatoren Porphyringemische unterschiedlichster Zusammensetzungen verwendet, die intravenös verabreicht werden müssen.

Die intravenöse Applikation derartiger Photosensibilisatoren birgt zwei entscheidende Nachteile in sich.

Zum einen führt sie zu einer Anreicherung der Porphyringemische in der gesamten Haut und löst damit bei Lichtexposition eine phototoxische Reaktion aus, die sich in Form von Rötung, Schwellung und Erosion der Haut sowie durch Schmerzen äußert. Die Patienten sind daher gezwungen, sich über mehrere Wochen vor starker Lichteinstrahlung zu schützen; praktisch bedeutet eine derartige Diagnose bzw. Therapie für den Betroffenen einen monatelangen Aufenthalt in verdunkelten Räumen!

Zum anderen schränkt die geringe Tumor-selektive Anreicherung der Porphyringemische die Effizienz eines Fluoreszenznachweises entscheidend ein. Diese Fluoreszenz-Differenz kann bisher nur mit aufwendigen computergestützten Bildanalysesystemen dargestellt werden, da sie mit bloßem Auge nicht sichtbar ist.

Des weiteren ist der therapeutische Nutzen intravenös appliziterter Photosensibilisatoren auch aufgrund der geringen Tumorselektivität eingeschränkt, und es wurden lokaltypische Nebenwirkungen, wie Schrumpfblasenbildng, beobachtet.

Es ist auch schon die Verwendung von δ-Aminolävulinsäure (ALA) zur Herstellung eines topischen Arzneimittels zur integralen Diagnose und/oder Therapie von Tumoren in Hohlorganen vorgeschlagen worden, vergl. DE-OS 42 28 106 und US-PS 5 079 262.

ALA ist als Vorläufer von Protoporphyrin IX bei der Bildung des Blutfarbstoffes (Hämbiosynthese) allgemein bekannt. Der bisherige klinische Einsatz von ALA beschränkte sich auf bestimmte Stoffwechselerkrankungen (Porphyrien), wo er sich jedoch als erfolglos erwies. So berichten Okuda et al. (Hepatology **1991**, 14, 1153-1160) von der Behandlung von Patienten mit Gilbert-Syndrom (Behcet-Krankheit) sowie von gesunden Probanden.

ALA ist als Substanz unbedenklich. Bei intravenöser Gabe von 0,23 mg ALA wurden keine toxischen Reaktionen beobachtet (Okuda et al., Hepatology **1991**, 14, 1153-1160, vgl. auch Kennedy et al., J. Photochem. Photobiol. B., **1990**, 6, 143-148). Nach Berlin et al. (Biochem. J. **1956**, 64, 80-90 und 90-100) wurden bei gesunden Probanden bei höherer Dosierung phototoxische Reaktionen lediglich für 1 bis 2 Tage beobachtet. Im Rahmen verschiedener Toxizitätsuntersuchungen an Tieren (Lima et al., Can. J. Neur. Sci. **1981**, 8, 105-114; Arnold et al., Food Cosmet. Toxicol. **1975**, 13, 63-68; Kennedy et al., ibid **1976**, 14, 45-48) konnten keine negativen Symptome hinsichtlich direkter Toxizität, Photosensitivität, Theratogenität oder subakuter Toxizität beobachtet werden.

Die in neuerer Zeit beschriebene topische Verwendung von ALA bei der Behandlung oberflächlicher Hauttumoren (vor allem Basaliome), vgl. Kennedy et al., (J. Photochem. Photobiol. B., **1990**, 6, 143-148), betrifft in erster Linie die visuell erkennbaren Tumoren, welche lokal gezielt mit der Substanz in Kontakt gebracht werden.

Die Wirkung von ALA beruht auf der Stimulierung der körpereigenen Porphyrinbildung. Der Verwendung von ALA gemäß DE-OS 42 28 106 liegt die Erkenntnis zugrunde, daß ALA bei topisch integraler Applikation auf die Schleimhäute von Hohlorganen selektiv von Tumor-Gewebe aufgenommen und angereichert wird und nur dort zu einer vermehrten Porphyrinbildung und -konzentration führt, während das gesunde Gewebe im wesentlichen unbeeinflußt bleibt. Es wurde gezeigt, daß der Anreicherungsfaktor gegenüber gesundem Gewebe bis zu 20 : 1 beträgt, mit dem Ergebnis, daß die Fluoreszenz der Tumor- bzw. Prätumor-Inseln selbst mit bloßem Auge erkennbar ist. Auf die früher notwendigen aufwendigen Bildverarbeitungssysteme kann dabei verzichtet werden.

Anders als bei den früheren Anwendungen wird ALA dabei nicht gezielt auf die zu behandelnden, bereits erkannten Tumoren aufgebracht, sondern es wird die ganze, das Hohlorgan auskleidende Schleimhaut homogen benetzt. Durch die tumorselektive Einlagerung von ALA fluoreszieren bei nachfolgender integraler Bestrahlung der Harnblase lediglich tumoröse Läsionen (Diagnose) bzw. werden zerstört (Therapie).

Gegenüber den älteren Verfahren zur Diagnose von Tumoren in Hohlorganen, die sich auf in fortgeschrittenen Stadien visuell sichtbare Tumoren beschränkten, besitzt die Verwendung von ALA entscheidende Vorteile. Die bei Instillation von ALA in Hohlorgane, wie z.B. die Harnblase, stattfindende selektive Anreicherung im Tumorgewebe drückt sich im Vergleich zu früheren Therapieansätzen in sehr viel höheren Differenzfaktoren gegenüber gesundem Gewebe aus. Während bei den intravenösen Applikationen von Porphyrin-Derivaten, die als undefinierte Gemische zur Diagnostik verwendet wurden, Differenzfaktoren von 2 bis 5 : 1 zwischen Tumor und gesundem Gewebe beobachtet wurden, beträgt der Differenzfaktor im Vergleich zu gesundem Gewebe bei der integralen Behandlung der Harnblase zum Beispiel 20 : 1. Durch die damit bei Bestrahlung verbundene starke Fluoreszenz tumorösen Gewebes gelingt erstmals die Diagnose präkanzeröser Läsionen, die bislang endoskopisch nicht erkennbar waren.

Die Nebenwirkungen einer Photosensibilisierung, die bei intravenöser Applikation von Photosensibilisatoren praktisch einen monatelangen Aufenthalt der Patienten in verdunkelten Räumen erforderlich machte, treten bei der Verwendung von ALA zur integralen Behandlung der Harnblase nicht auf. Ferner kann auf komplizierte Bildverarbeitungssysteme zur Diagnostik infolge des verbesserten Differenzfaktors verzichtet werden.

Durch die Stimulierung der körpereigenen Porphyrinbildung infolge der tumorselektiven Absorption bei der Verwendung von ALA können nicht nur Blasenkarzinome im Frühstadium erkannt werden (integrale Fluoreszenzmarkierung), sondern es können durch nachfolgende integrale Bestrahlung der Harnblase selektiv die tumorösen Läsionen zerstört werden (integrale Therapie). Damit an sich steht erstmals eine Therapie für präkanzeröse und kanzeröse Läsionen zur Verfügung, die bislang aufgrund der fehlenden Möglichkeit zur Diagnosestellung in frühen Krankheitsstadien nicht therapiert wurden. Die bislang für z.B. Blasentumoren beobachteten Rezidiv- und Progressionsraten lassen sich durch die Verwendung von ALA zur Diagnose und/oder Therapie entscheidend verringern.

Gemäß US-PS 5 079 262 werden topisch hochkonzentrierte ALA-Lösungen mit einem Wirkstoffgehalt zwischen 10 und 33 Gew.% angewendet. Derartige ALA-Lösungen sind selbst bei Herstellung unmittelbar vor der Anwendung nicht stabil und zersetzen sich in kürzester Zeit.

In der DE-OS 42 28 106 wird vorgeschlagen, zur diagnostischen Anwendung ALA in 3%-iger wässriger Lösung anzuwenden, welche mittels Natriumbicarbonat als Puffer auf einen neutralen pH eingestellt ist. Für therapeutische Anwendungen soll die Konzentration der neutral gepufferten ALA-Lösung bis zu 10% betragen. Es hat sich jedoch gezeigt, daß auf den physiologischen pH-Wert von etwa 7 eingestellte ALA-Lösungen nicht stabil sind. Schon bei einem pH-Wert über 5,2 findet eine Zersetzung des Wirkstoffes unter gleichzeitiger Gelbfärbung der Lösung statt. Andererseits führen Lösungen von δ-Aminolävulinsäurehydrochlorid, welche schon bei einer Konzentration von 1% einen pH-Wert zwischen 2,2 und 3,2 aufweisen, zu einem starken, für den Patienten schwer erträglichen Brennen.

Es wurde nunmehr gefunden, daß der pH-Wert der ALA-Lösung zwischen 4,8 und 5,2 liegen muß, um sowohl eine gute Verträglichkeit als auch eine ausreichende Stabilität zu erreichen. Ein pH-Wert oberhalb 5,2 führt zur Instabilität der δ-Aminolävulinsäure, bei einem pH-Wert unterhalb 4,8 verschlechtert sich das Nebenwirkungsprofil signifikant. Zur Herstellung der ALA-Lösung wird die physiologisch verträgliche Pufferlösung vorzugsweise erst kurz vor der Anwendung zu dem sterilen ALA-Lyophilisat hinzugegeben. Dabei treten zwangsläufig gewisse Konzentrationsschwankungen auf, welche in einer Größenordnung von ± 10% liegen können. Dies bedeutet mit anderen Worten, daß bei der Herstellung einer 3%-igen Lösung der tatsächliche Gehalt zwischen 2,7 und 3,3% schwanken kann. Es wurde gefunden, daß insbesondere mit Hilfe von Natriumhydrogenphosphat (Na₂HPO₄ · 12 H₂O) die Einstellung auf den gewünschten pH-Wertbereich von 4,8 bis 5,2 gelingt, während dies mit anderen physiologisch an sich brauchbaren Puffern wie Natriumbicarbonat nicht ohne weiteres möglich ist.

Gegenstand der Erfindung ist demgemäß ein topisch anwendbares Arzneimittel zur integralen Diagnose und/oder Therapie von Tumoren in Hohlorganen auf Basis von δ-Aminolävulinsäure, wobei das Arzneimittel dadurch gekennzeichnet ist, daß es δ-Aminolävulinsäure in einer wässrigen Lösung enthält, welche durch eine physiologisch verträgliche Pufferlösung auf einen pH-Wert von 4,8 bis 5,2 eingestellt ist. Vorzugsweise wird der pH-Wert durch eine physiologisch verträgliche Säure und Natriumhydrogenphosphat auf einen pH-Wert von 4,8 bis 5,2 eingestellt. Die physiologisch verträgliche Säure ist vorzugsweise Salzsäure. Eine besonders vorteilhafte Ausführungsform der Erfindung besteht darin, daß 6-Aminolävulinsäure als Hydrochlorid eingesetzt wird.

Günstigerweise wird die Arzneimittellösung erst unmittelbar vor der Anwendung aus δ-Aminolävulinsäurehydrochlorid in Form eines Lyophilisats unter Zugabe der Natriumhydrogenphosphat-Pufferlösung hergestellt, wobei das Arzneimittel dem Arzt in Form einer Kombinationspackung zur Verfügung gestellt wird. Vorzugsweise handelt es sich bei der Kombinationspackung bereits um die Teile einer Anordnung, welche bei Gebrauch zur Instillation des Arzneimittels dienen können.

Das Arzneimittel ist für die Diagnose und/oder Therapie des Urothels und des Zentralnervensystems (bevorzugt der verschiedenen Hirnareale) sowie für die integrale Diagnose und/oder Therapie von Hohlorganen wie der Harnblase (Harnwege), der Atemwege, der Peritonealhöhle, des Gastrointestinaltraktes, oder des weiblichen Genitialtrakts geeignet, wobei die Harnblase und der weibliche Genitialtrakt bevorzugt in Betracht kommen.

Die Applikation kann - beispielsweise bei der Harnblase-entweder topisch intravesikal erfolgen oder in einer bevorzugten Ausführungsform durch Instillation der ALA-Lösung geschehen.

Zur Diagnose wird das Hohlorgan bzw. das betroffene Areal mit Licht der Wellenlänge 390 bis 430 nm, vorzugsweise 410 nm, integral bestrahlt, so daß tumoröse Läsionen fluoreszieren. Vorzugsweise kann durch Filter gebrochenes Licht einer Wellenlänge von 406 nm verwendet werden. Zur integralen diagnostischen Bestrahlung ist ein Krypton-Ionen-Laser besonders geeignet.

Um die tumorösen Läsionen zu zerstören, wird nach Instillation des erfindungsgemäßen Arzneimittels das betroffene Areal flächig bzw. das Hohlorgan integral mit Licht der Wellenlänge 600 bis 650 nm, vorzugsweise 630 nm, bestrahlt. Insbesondere kommt bei der therapeutischen lokalen Bestrahlung ein Nd:Yag-Laser (Neodym:Yttrium-Aluminium-Garnet-Laser) zum Einsatz.

Die Erfindung wird nachfolgend anhand des Beispiels erläutert.

### Beispiel

Es wurde eine sterile Lösung von 5,4 Gew.% Na₂HPO₄ · 12H₂O in sterilem Wasser hergestellt. Der pH-Wert dieser Phosphatlösung betrug ca. 9.

Zu 100 ml dieser Pufferlösung wurden 3 g δ-Aminolävulinsäurehydrochlorid gegeben. Der pH-Wert stellt sich dadurch auf 4,85 ein. Die Osmolalität dieser Lösung betrug 700 mOsm/kg. Die Lösung zeigte auch nach mehrstündigem Stehen bei Raumtemperatur keine Verfärbung, während eine Lösung von pH > 5,2 sich innerhalb von Minuten gelblich verfärbt; bei einem pH-Wert > 6 setzt die Verfärbung deutlich sichtbar schon bei der Bildung der Lösung ein, nach einstündigem Stehen ist eine derartige Lösung stark orange-gelb verfärbt. Weitere Versuche ergaben, daß der pH-WertBereich von 4,8 bis 5,2 mit Hilfe der 5,4%-igen Natriumhydrogenphosphatlösung auch dann noch eingehalten werden kann, wenn die ALA-Konzentration um ± 10% variiert, d.h. zwischen 2,7 und 3,3 Gew.% liegt.

Die 3%-ige ALA-Lösung mit einem pH-Wert von 4,85 wurde bei Instillation in die Blase von Patienten gut vertragen. Insbesondere trat kein unangenehmes Brennen auf, wie dies bei ungepufferten ALA-Lösungen beoachtet wird.

## Patentansprüche

1. Topisch anwendbares Arzneimittel zur integralen Diagnose und/oder Therapie von Tumoren in Hohlorganen auf Basis von δ-Aminolävulinsäure, dadurch gekennzeichnet, daß es 6-Aminolävulinsäure in einer wässrigen Lösung enthält, welche durch eine physiologisch verträgliche Säure und Natriumhydrogenphosphat auf einen pH-Wert von 4,8 bis 5,2 eingestellt ist.

2. Arzneimittel gemäß Anspruch 1, dadurch gekennzeichnet, daß es Salzsäure als Säure enthält.

3. Arzneimittel gemäß den Ansprüchen 1 oder 2, dadurch gekennzeichnet, daß es die δ-Aminolävulinsäure als Hydrochlorid enthält.

4. Arzneimittel gemäß einem der Ansprüche 1 bis 3, dadurch gekennzeichnet, daß es für die Herstellung unmittelbar vor Gebrauch in Form einer Verpackungseinheit aus lyophilisiertem δ-Aminolävulinsäurehydrochlorid einerseits und der Natriumhydrogenphosphatlösung andererseits vorliegt.

5. Arzneimittel gemäß einem der Ansprüche 1 bis 4, dadurch gekennzeichnet, daß es für die Instillation formuliert ist.

## Claims

1. Topically-applicable medicament for the integral diagnosis and/or therapy of tumours in hollow organs based on delta-aminolevulinic acid, characterised in that it contains delta-aminolevulinic acid in an aqueous solution which is adjusted to a pH value of 4.8 to 5.2 by means of a physiologically tolerable acid and sodium hydrogen phosphate.

2. Medicament in accordance with Claim 1, characterised in that it contains hydrochloric acid as the acid.

3. Medicament in accordance with Claims 1 or 2, characterised in that it contains the delta-aminolevulinic acid in the form of a hydrochloride.

4. Medicament in accordance with one of Claims 1 to 3, characterised in that it is provided in the form of a packaging unit of lyophilised delta-aminolevulinic acid hydrochloride and the sodium hydrogen phosphate solution, for manufacture immediately before use.

5. Medicament in accordance with one of Claims 1 to 4, characterised in that it is formulated for instillation.

## Revendications

1. Médicament pour application locale pour le diagnostic et/ou le traitement intégraux de tumeurs des organes creux à base de l'acide δ-aminolévulinique caractérisé par le fait qu'il contient de l'acide δ-aminolévulinique dans une solution aqueuse dont le pH est compris entre 4,8 et 5,2 grâce à l'ajout d'un acide physiologiquement supportable et d'hydrogénophosphate de sodium.

2. Médicament selon la revendication 1 caractérisé par le fait qu'il contient de l'acide chlorhydrique comme acide.

3. Médicament selon la revendication 1 ou 2 caractérisé par le fait qu'il contient de l'acide δ-aminolévulinique sous la forme d'hydrochlorure.

4. Médicament selon les revendications 1 à 3 caractérisé par le fait qu'il est fourni pour fabrication immédiatement avant utilisation sous la forme d'un emballage contenant de l'hydrochlorure d'acide δ-aminolévulinique d'une part et la solution d'hydrogénophosphate de sodium d'autre part.

5. Médicament selon les revendications 1 à 4 caractérisé par le fait qu'il est formulé pour instillation.
